# EUROPEAN PATENT APPLICATION

(11) **EP 4 389 121 A1**
(43) Date of publication of application: **26.06.2024**
(21) Application number: 22383267.6
(22) Date of filing: 22.12.2022
(51) Int. Cl.: A61K 31/197, A61P 31/14, C07D 409/00, A61K 31/445, A61K 31/4523, A61K 31/4535, A61K 31/47

(54) **ANTIVIRAL AMINO ACID DERIVATIVES**

(71) Applicant: Consejo Superior De Investigaciones Científicas (CSIC), 28006 Madrid (ES)
(72) Inventor: Rodríguez Franco, María Isabel, 28006 MADRID (ES); Garaigorta de Dios, Urtzi, 28049 MADRID (ES); Gastaminza Landart, Pablo, 28049 MADRID (ES)
(74) Representative: Pons

(57) **Abstract**

The present invention relates to an amino acid derivative of formula I or a pharmaceutically acceptable salt for use in the treatment and/or prevention of a viral disease caused by a coronavirus, particularly SARS-CoV-2 or HCoV 229E, wherein the R₁, R₂ and R₃ groups have the meaning described in the description.

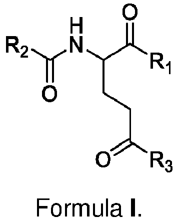

## Description

The present invention relates to an amino acid derivative of formula **I** or a pharmaceutically acceptable salt thereof for use in the treatment and/or prevention of a viral disease caused by a coronavirus, particularly SARS-CoV-2 or HCoV 229E. Therefore, the invention may be comprised in the field of medical chemistry and, specifically, in the field of antivirals.

### STATE OF THE ART

Human coronaviruses can cause mild as well as severe respiratory symptoms in humans. The emergence in the last two decades of three new coronaviruses (i.e. severe acute respiratory syndrome coronaviruses 1 (SARS-CoV-1, Peiris JS, Yuen KY, Osterhaus AD, Stöhr K. The severe acute respiratory syndrome. N Engl J Med. 2003 Dec 18;349(25):2431-41)] and 2 (SARS-CoV-2, Zhou P, Yang XL, Wang XG, Hu B, Zhang L, Zhang W, Si HR, Zhu Y, Li B, Huang CL, Chen HD, Chen J, Luo Y, Guo H, Jiang RD, Liu MQ, Chen Y, Shen XR, Wang X, Zheng XS, Zhao K, Chen QJ, Deng F, Liu LL, Yan B, Zhan FX, Wang YY, Xiao GF, Shi ZL. A pneumonia outbreak associated with a new coronavirus of probable bat origin. Nature. 2020 Mar;579(7798):270-273) and Middle East respiratory syndrome coronavirus (MERS-CoV, see for exameple Zumla A, Hui DS, Perlman S. Middle East respiratory syndrome. Lancet. 2015 Sep 5;386(9997):995-1007)) with potential for causing devastating pandemics has revealed the need for preparedness against new emergent and re-emergent pathogens, including current and new coronaviruses.

Despite the rapid development of preventive vaccines against coronaviruses, new escape virus variants continue to emerge and therefore therapeutics are still required to fight these viral infections. In the last years several agents have been approved by the regulatory agencies for the treatment or prevention of diseases caused by coronavirus infection. The first agent approved was remdesivir (commercialized as Veklury), that was shown to interfere with the RNA polymerization step of coronavirus infections in cell culture and was further moved into clinical practice for preventing the development of severe forms of coronavirus disease infection in humans. However, its efficacy and usefulness in the clinical setting is limited (Grein J, Ohmagari N, Shin D, Diaz G, Asperges E, Castagna A, Feldt T, Green G, Green ML, Lescure FX, Nicastri E, Oda R, Yo K, Quiros-Roldan E, Studemeister A, Redinski J, Ahmed S, Bernett J, Chelliah D, Chen D, Chihara S, Cohen SH, Cunningham J, D'Arminio Monforte A, Ismail S, Kato H, Lapadula G, L'Her E, Maeno T, Majumder S, Massari M, Mora-Rillo M, Mutoh Y, Nguyen D, Verweij E, Zoufaly A, Osinusi AO, DeZure A, Zhao Y, Zhong L, Chokkalingam A, Elboudwarej E, Telep L, Timbs L, Henne I, Sellers S, Cao H, Tan SK, Winterbourne L, Desai P, Mera R, Gaggar A, Myers RP, Brainard DM, Childs R, Flanigan T. Compassionate Use of Remdesivir for Patients with Severe Covid-19. N Engl J Med. 2020 Jun 11;382(24):2327-2336).

Another direct acting antiviral agent approved for the treatment of coronavirus infections in humans is commercialized under paxlovid brand name. This agent is a combination of nirmatrelvir (a coronavirus main protease inhibitor) and ritonavir. It is an orally-given drug able to decrease coronavirus replication (Akinosoglou K, Schinas G, Gogos C. Oral Antiviral Treatment for COVID-19: A Comprehensive Review on Nirmatrelvir/Ritonavir. Viruses. 2022 Nov 17;14(11):2540).

Other approved treatments against coronavirus infections are monoclonal antibody-based therapies directed against the viral spike protein, that prevent viral entry into the cells (e.g. regdanvimab, casirivimab, indevimab and sotrovimab). Because of the intrinsic variability of the spike protein these treatments might not be effective against emergent virus variants. Another monoclonal antibody-based approved therapy known as tocilizumab is directed against a secreted cytokine (interleukin 6) involved in the cytokine storm. Thus, this treatment prevents the inflammatory response to the infection, but has no impact on viral replication.

From what is disclosed in the art, there is still a need for the generation of small molecule antiviral drugs to be used in the treatment or prevention of infection by coronaviruses.

### DESCRIPTION OF THE INVENTION

A first aspect of the invention relates to a compound of formula I 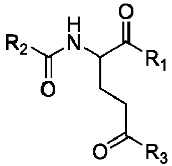 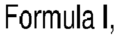 or a pharmaceutically acceptable salt thereof, wherein:
R₁ represents an -OH, -OR₄ or -NHR₅ group;
R₂ represents a C₁-C₆ alkyl group optionally substituted by phenyl, an -OCy₁ or Cy₁ group;
R₃ represents an -OH, -OR₄ or -NHR₅ group;
R₄ represents a C₁-C₆ alkyl optionally substituted by a halogen group;
R₅ represents a C₁-C₆ alkyl optionally substituted by a Cy₂ group;
Cy₁ represents a 5 or 6-membered aromatic ring that is optionally fused to another 5 or 6-membered aromatic ring and optionally contains 1 or 2 heteroatoms selected from N, O and S, and is optionally substituted by one or more C₁-C₆ alkyl or -O-C₁-C₆ alkyl groups; and
Cy₂ represents a 5 or 6 membered saturated heterocycle containing 1 or 2 heteroatoms selected from N, O and S, and is optionally substituted by a C₁-C₆ alkyl group which in turn is optionally substituted by a phenyl group,
for use in the treatment and/or prevention of a viral disease caused by a coronavirus, preferably wherein the coronavirus is SARS-CoV-2, HCoV 229E, HCoV NL63, HCoV HKU1, HCoV OC43, SARS-CoV-1 or MERS-CoV, and more preferably wherein the coronavirus is SARS-CoV-2 or HCoV 229E.

In another embodiment, the invention relates to a compound of formula I or a pharmaceutically acceptable salt thereof for the use defined above, wherein R₁ represents -OR₄ or -NHR₅ group, and preferably an -OR₄ group.

In another embodiment, the invention relates to a compound of formula I or a pharmaceutically acceptable salt thereof for the use defined above, wherein:
R₁ represents -OR₄ or -NHR₅ group, and preferably an -OR₄ group; and
R₄ represents a C₁-C₆ alkyl optionally substituted by a Br group.

In another embodiment, the invention relates to a compound of formula I or a pharmaceutically acceptable salt thereof for the use defined above, wherein:
R₁ represents -OR₄ or -NHR₅ group, and preferably an -OR₄ group; and
R₄ represents a C₁-C₆ alkyl.

In another embodiment, the invention relates to a compound of formula I or a pharmaceutically acceptable salt thereof for the use defined above, wherein R₁ represents an -NHR₅ group.

In another embodiment, the invention relates to a compound of formula I or a pharmaceutically acceptable salt thereof for the use defined above, wherein:
R₁ represents an -NHR₅ group; and
R₅ represents a C₁-C₆ alkyl optionally substituted by a Cy₂ group;

In another embodiment, the invention relates to a compound of formula I or a pharmaceutically acceptable salt thereof for the use defined above, wherein:
R₁ represents an -NHR₅ group;
R₅ represents a C₁-C₆ alkyl optionally substituted by a Cy₂ group; and Cy₂ represents *N*-benzylpyridine;

In another embodiment, the invention relates to a compound of formula I or a pharmaceutically acceptable salt thereof for the use defined above, wherein R₂ represents a C₁-C₆ alkyl group substituted by phenyl, an -OCy₁ or Cy₁ group.

In another embodiment, the invention relates to a compound of formula I or a pharmaceutically acceptable salt thereof for the use defined above, wherein R₂ represents a C₁-C₆ alkyl group substituted by phenyl.

In another embodiment, the invention relates to a compound of formula I or a pharmaceutically acceptable salt thereof for the use defined above, wherein:
R₂ represents a C₁-C₆ alkyl group substituted by an -OCy₁ or Cy₁ group; and
Cy₁ represents a 5 or 6-membered aromatic ring that is optionally fused to another 5 or 6-membered aromatic ring and optionally contains 1 or 2 heteroatoms selected from N, O and S, and is optionally substituted by one or more C₁-C₆ alkyl or -O-C₁-C₆ alkyl groups.

In another embodiment, the invention relates to a compound of formula I or a pharmaceutically acceptable salt thereof for the use defined above, wherein R₃ represents -OR₄ or -NHR₅ group, and preferably wherein R₃ represents an -NHR₅ group.

In another embodiment, the invention relates to a compound of formula I or a pharmaceutically acceptable salt thereof for the use defined above, wherein:
R₃ represents an -NHR₅ group; and
R₅ represents a C₁-C₆ alkyl optionally substituted by a Cy₂.group.

In another embodiment, the invention relates to a compound of formula I or a pharmaceutically acceptable salt thereof for the use defined above, wherein Cy₂ represents a heterocycle of formula Cy₂ₐ:

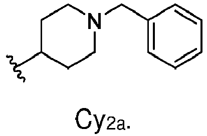

In another embodiment, the invention relates to a compound of formula I or a pharmaceutically acceptable salt thereof for the use defined above, wherein:
R₁ represents an -NHR₅ group;
R₅ represents a C₁-C₆ alkyl optionally substituted by a Cy₂ group; and Cy₂ represents Cy₂ₐ;

In another embodiment, the invention relates to a compound of formula I or a pharmaceutically acceptable salt thereof for the use defined above, wherein:
R₃ represents an -NHR₅ group; and
R₅ represents C₁-C₆ alkyl optionally substituted by a Cy₂ₐ.group.

In another embodiment, the invention relates to a compound of formula I or a pharmaceutically acceptable salt thereof for the use defined above, wherein R₃ represents a group of formula R₃ₐ:

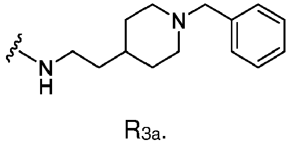

In another embodiment, the invention relates to a compound of formula I or a pharmaceutically acceptable salt thereof for the use defined above, wherein:
R₁ represents -OR₄ or -NHR₅ group, and preferably an -OR₄ group; and
R₂ represents a C₁-C₆ alkyl group substituted by phenyl, an -OCy₁ or Cy₁ group;
R₃ represents an -NHR₅ group, and preferably a group of formula R₃ₐ;
R₄ represents a C₁-C₆ alkyl which is optionally substituted by a halogen group, and preferably by a Br group.

In another embodiment, the invention relates to a compound of formula I or a pharmaceutically acceptable salt thereof for the use defined above, wherein the compound of formula I is a compound of formula **I**-L: 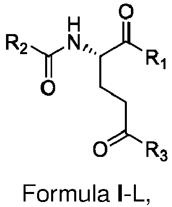 wherein:
R₁ represents an -OH, -OR₄ or -NHR₅ group;
R₂ represents a C₁-C₆ alkyl group optionally substituted by phenyl, an -OCy₁ or Cy₁ group;
R₃ represents an, -OH, -OR₄ or -NHR₅ group;
R₄ represents a C₁-C₆ alkyl optionally substituted by a halogen group;
R₅ represents a C₁-C₆ alkyl optionally substituted by a Cy₂ group;
Cy₁ represents a 5 or 6-membered aromatic ring that is optionally fused to another 5 or 6-membered aromatic ring and optionally contains 1 or 2 heteroatoms selected from N, O and S, and is optionally substituted by one or more C₁-C₆ alkyl or -O-C₁-C₆ alkyl groups; and
Cy₂ represents a 5 or 6 membered saturated heterocycle containing 1 or 2 heteroatoms selected from N, O and S, and is optionally substituted by a C₁-C₆ alkyl group which in turn is optionally substituted by a phenyl group.

In another embodiment, the invention relates to a compound of formula I or a pharmaceutically acceptable salt thereof for the use defined above, wherein the compound of formula **I** is selected from:

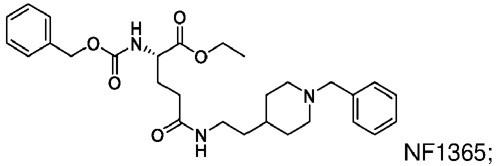

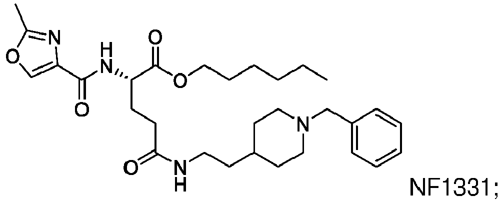

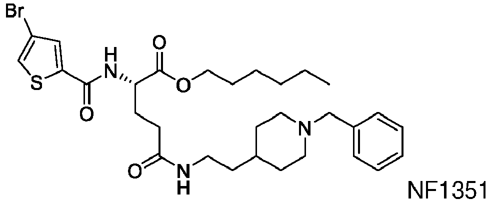

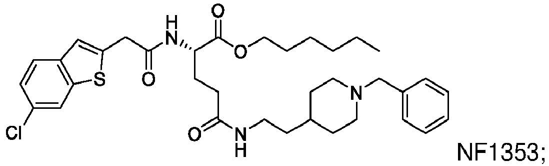

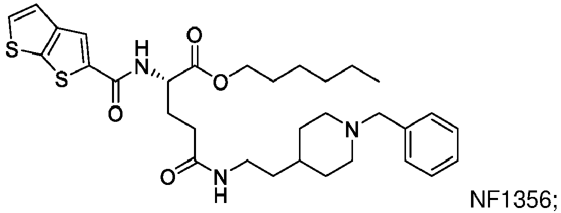

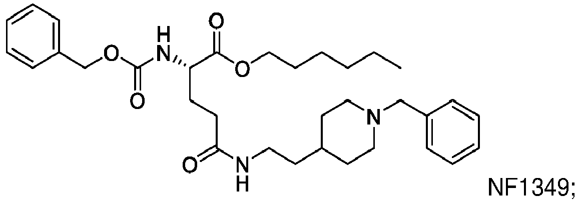

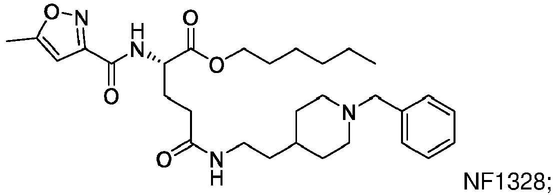

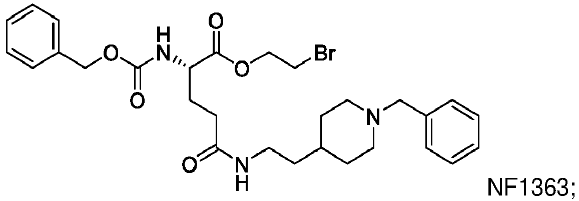

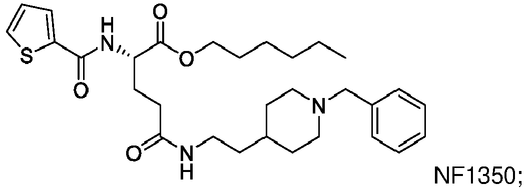

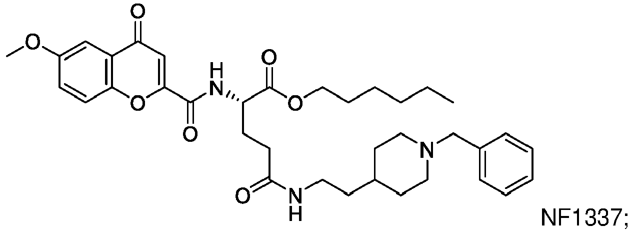

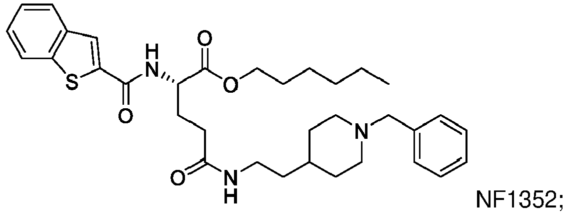

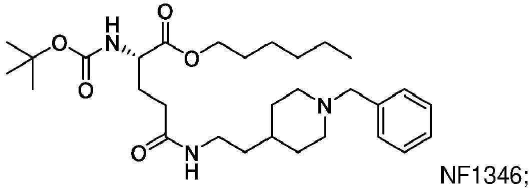

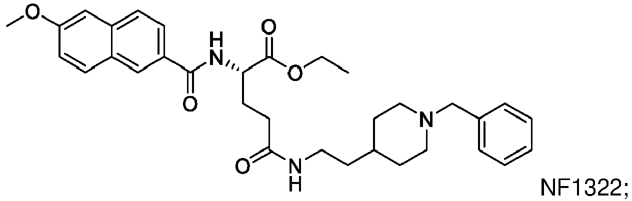

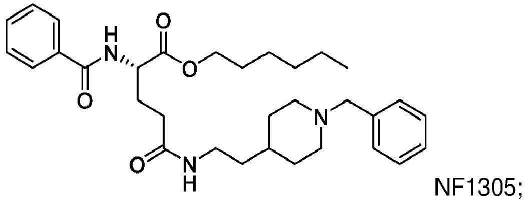

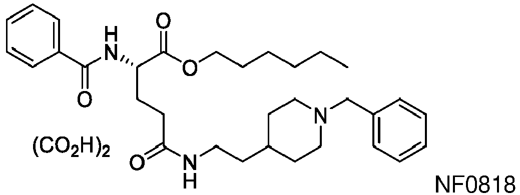

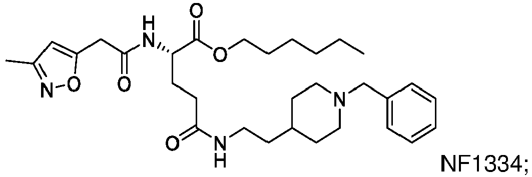

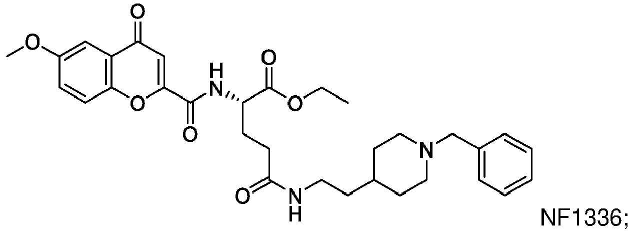

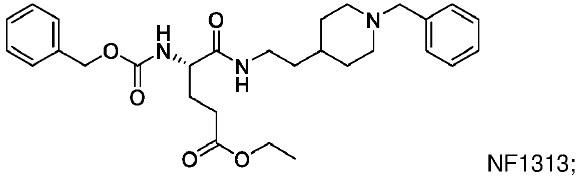

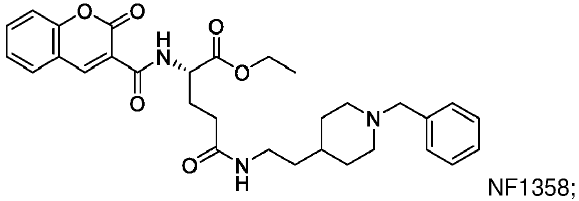

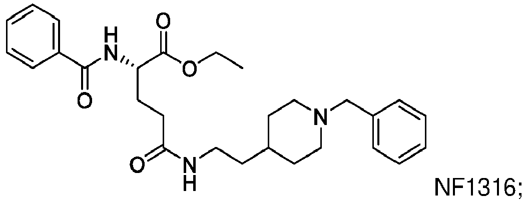

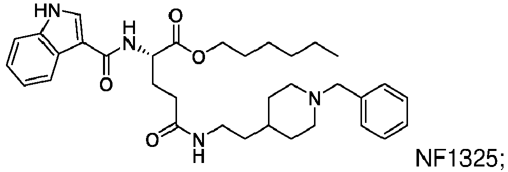

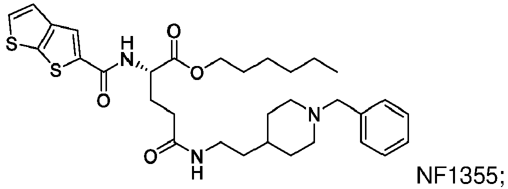

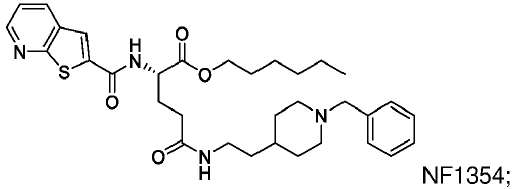

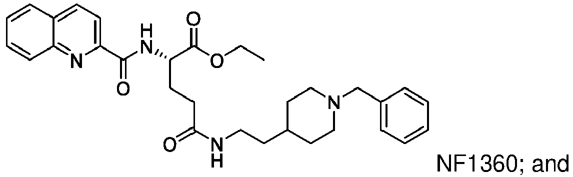

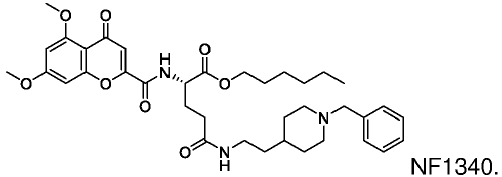

In another embodiment, the invention relates to a compound of formula **I** or a pharmaceutically acceptable salt thereof for the use defined above, wherein the compound of formula **I** is selected from NF1331, NF1365 and NF1349.

The compounds of the invention show selectivity towards members of the *Coronoviridae* family of viruses, such as SARS-CoV-2 and HCoV 229E. In contrast, they do not show any antiviral activity against other family of viruses including: Flaviviruses, that contain a positive stranded RNA genome (i.e. West Nile virus) and Rhabdoviruses, that contain a negative stranded RNA genome (i.e. Vesicular Stomatitis virus).

Another aspect of the invention relates to a compound of formula **I** selected from:

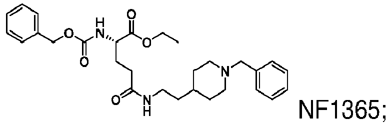

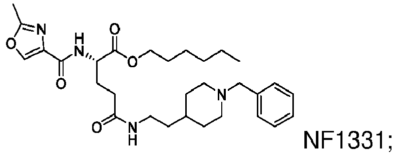

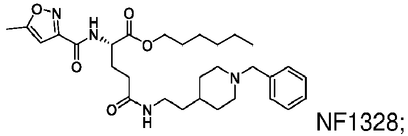

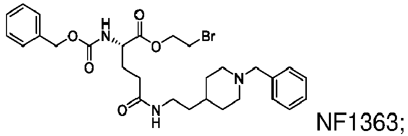

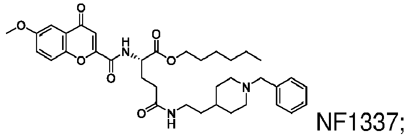

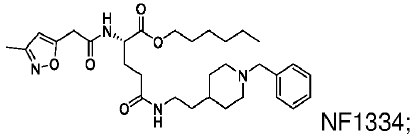

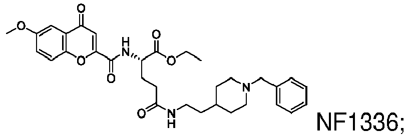

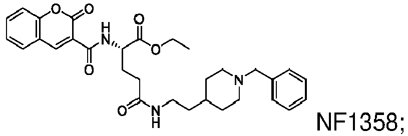

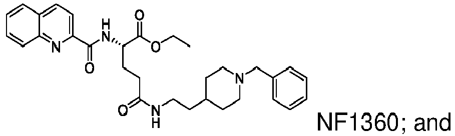

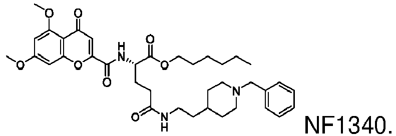

Another aspect of the invention relates to a compound of formula **I** selected from: 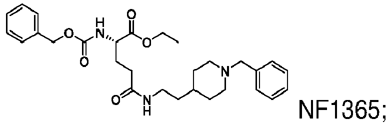 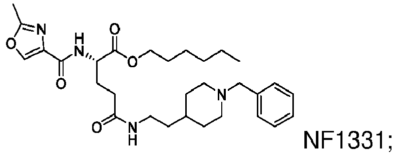 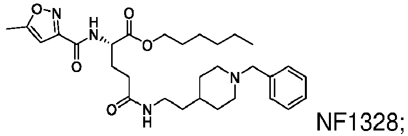 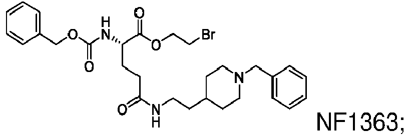 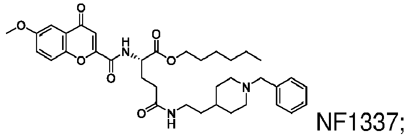 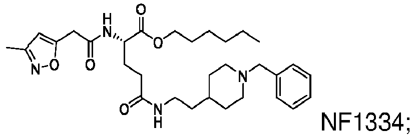 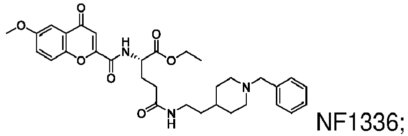 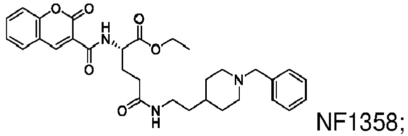 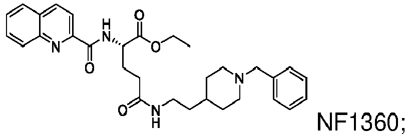 and 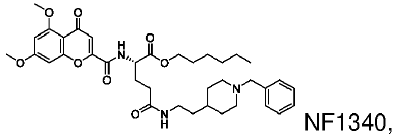 or a pharmaceutically acceptable salt thereof for use as a medicament.

Another aspect of the invention relates to the use of a compound of formula **I** selected from: 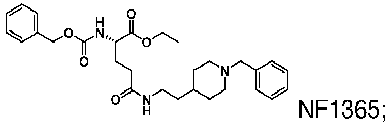 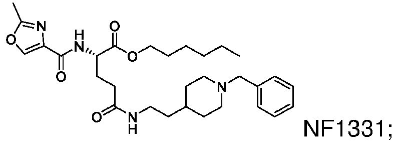 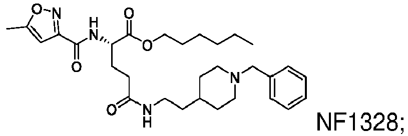 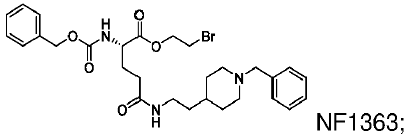 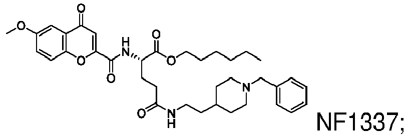 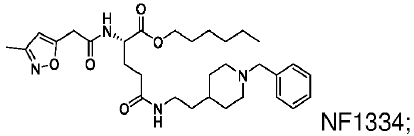 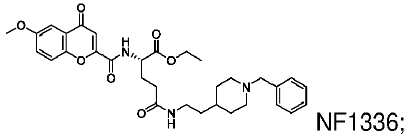 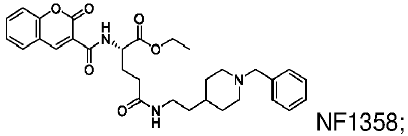 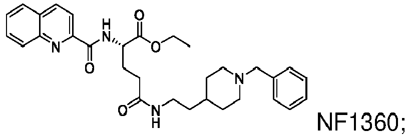 and 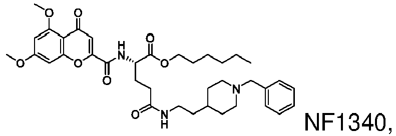 or a pharmaceutically acceptable salt thereof, for manufacturing a medicament.

Another aspect of the invention relates to a method for the treatment and/or prevention of a disease in a subject in need thereof, particularly in humans, which comprises administering to said subject an effective amount of a compound of formula I selected from: 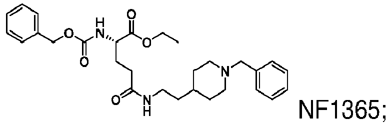 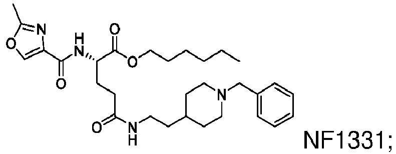 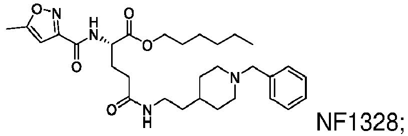 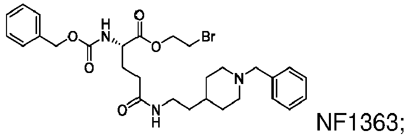 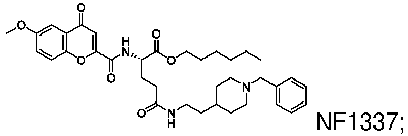 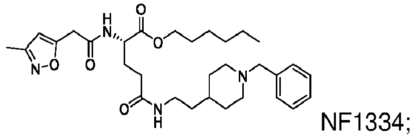 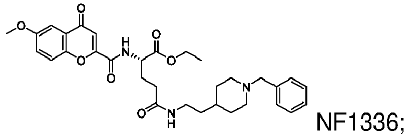 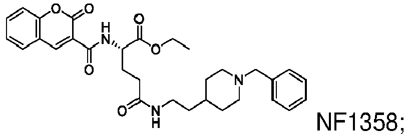 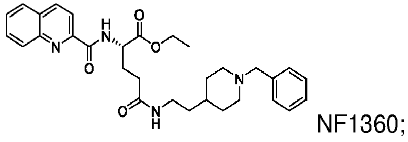 and 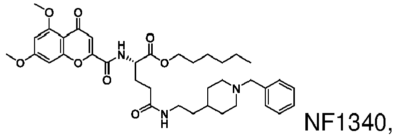 or a pharmaceutically acceptable salt thereof.

Throughout the description and claims of the present invention, a halogen group or its abbreviation halo means fluoro, chloro, bromo or iodo.

The term C₁₋₆ alkyl, as a group or part of a group, means a straight or branched chain alkyl group containing from 1 to 6 C atoms. Examples include, among others, methyl, ethyl, propyl, isopropyl, butyl, isobutyl, sec-butyl, terc-butyl, pentyl and hexyl groups.

Where in the definitions used throughout this description for cyclic groups, such as for example Cy₁, Cy₂ and Cy₂ₐ, the specified examples refer to a radical of a ring in general terms, with all the possible binding positions being included.

The expression "optionally substituted by one or more groups" means the possibility of a group being substituted by one or more, preferably by 1, 2, 3 or 4 substituents, more preferably with 1, 2 or 3 substituents, and even more preferably with 1 or 2 substituents, provided that said group has enough positions available for substitution. If they are present, said substituents may be the same or different and may be located on any available position.

The term "optionally substituted by a group" means the possibility of a group being substituted by another substituent group located on any available position.

Throughout the present description, the term "treatment" refers to eliminating, reducing or lessening the cause or effects of a disease. For the purposes of this invention, treatment includes, but is not limited to, alleviating, lessening or eliminating one or more symptoms of the disease, reducing disease intensity, stabilizing (i.e., not worsening) the state of the disease, delaying or slowing the progression of the disease, alleviating or improving the state of the disease and causing (either total or partial) remission.

As it is used herein, the term "prevention" refers to preventing the onset of disease from occurring in a patient who is predisposed or has risk factors, but still has not shown any symptoms of the disease. Prevention also includes preventing the recurrence of a disease in a subject who has previously suffered from said disease.

As it is used herein, the term "coronavirus" refers to members of the family *Coronaviridae* as defined by the International Committee on Taxonomy of Viruses (ICTV), that currently includes 7 human coronaviruses such as: HCoV 229E, HCoV NL63, HCoV HKU1, HCoV OC43, SARS-CoV-1, MERS-CoV and SARS-CoV-2.

The compounds of the present invention contain one or more basic nitrogen and could therefore form salts with acids, both organic and inorganic. Some compounds of the present invention may contain one or more acid protons and could therefore also form salts with bases. Examples include among others, oxalate ((CO₂H)₂, see example NF 0818), maleate, citrate, tartrate, gluconate, mesylate, acetate, sulfate, nitrate and hydrochloride.

There is no limitation to the type of salt that can be used, with the condition that when used for therapeutic purposes they are pharmaceutically acceptable. Pharmaceutically acceptable salts are understood to be those salts that, according to medical criterion, are suitable for use in contact with human or other mammal tissues without causing undue toxicity, irritation, allergic response or the like. Pharmaceutically acceptable salts are widely known to any person skilled in the art.

The salts of a compound of formula **I** can be obtained during the final isolation and purification of the compounds of the invention or can be prepared by reacting a compound of formula **I** with a sufficient amount of the desired acid or base to give the salt as a conventional shape. The salts of the compounds of formula **I** can be transformed in turn into other salts of compounds of formula **I** by ion exchange by means of an ion exchange resin.

The compounds of formula **I** and their salts may differ in certain physical properties, but are equivalent for the purposes of the invention. All the salts of the compounds of formula **I** are included within the scope of the invention.

The compounds of the present invention may form complexes with solvents wherein they are made to react or precipitate or crystallize. These complexes are known as solvates. As used herein, the term solvate refers to a complex of variable stoichiometry formed by a solute (a compound of formula **I** or a salt thereof) and a solvent. Examples of solvents include pharmaceutically acceptable solvents such as water, ethanol and the like. A complex with water is known as a hydrate. The solvates of the compounds of the invention (or their salts), including hydrates, are included within the scope of the invention.

The compounds of formula **I** may exist in different physical forms, i.e., amorphous, and crystalline forms. Likewise, the compounds of the present invention may be capable of crystallizing into more than one form, a characteristic known as polymorphism. Polymorphs can be differentiated by various physical properties well known to persons skilled in the art, such as for example their X-ray diffractograms, solubility or melting points. All the physical forms of compounds of formula **I,** including all their polymorphic forms ("polymorphs"), are included within the scope of the present invention.

Some compounds of the present invention could exist in the form of various diastereoisomers and/or various optical isomers. Diastereoisomers can be separated using conventional techniques such as chromatography or fractionated crystallization. Optical isomers can be resolved using conventional optical resolution techniques, to give optically pure isomers. This resolution can be performed on the synthetic intermediates that are chiral or on the products of formula **I**. The optically pure isomers can also be obtained individually using enantio-specific syntheses. The present invention covers both the individual isomers and their mixtures (for example, racemic mixtures or mixtures of diastereoisomers), whether obtained by synthesis or physically mixing them.

The present invention also relates to a pharmaceutical composition comprising a compound of the invention (or a pharmaceutically acceptable salt or solvate thereof) and one or more pharmaceutically acceptable excipients. Excipients must be "acceptable" in the sense of being compatible with the other ingredients of the composition and of not being harmful to those taking said composition.

The compounds of the present invention may be administered in the form of any pharmaceutical composition, whose nature, as is well known, will depend on the nature of the active ingredient and on its administration route. In principle, any administration route may be used, for example oral, parenteral, nasal, ocular, rectal, and topical.

Solid compositions for oral administration include tablets, granulates and capsules. In any case, the manufacturing method is based on simple mixture, dry granulation or wet granulation of the active ingredient with excipients. These excipients can be, for example, diluents such as lactose, microcrystalline cellulose, mannitol or calcium hydrogen phosphate; binding agents such as, for example, starch, gelatin or polyvinylpyrrolidone; desintegrants such as sodium carboxymethyl starch or sodium croscarmellose; and lubricating agents such as, for example, magnesium stearate, stearate acid or talcum. The tablets may also be coated with suitable excipients and by means of known techniques for the purpose of delaying their disaggregation and absorption in the gastrointestinal tract, thereby achieving sustained action for a longer period of time, or simply for improving their organoleptic properties or their stability. The active ingredient may also be incorporated by coating of inert pellets using natural or synthetic filmogenic polymers. The manufacture of soft gelatin capsules is also possible, wherein the active ingredient is mixed with water or with an oily medium, for example, coconut oil, liquid paraffin or olive oil.

Powders and granulates for the preparation of oral suspensions can be obtained by adding water, mixing the active ingredient with dispersing or wetting agents; suspensates and preservatives. Other excipients may also be added, for example, sweeteners, flavorings and colorants.

Liquid forms for oral administration may include emulsions, solutions, suspensions, syrups and elixirs containing commonly used inert diluents, such as distilled water, ethanol, sorbitol, glycerol, polyethylene glycols (macrogols) and propylene glycol. Said compositions may also contain coadjuvants such as wetting agents, suspensates, sweeteners, flavorings, preservatives, and pH regulators.

Injectable preparations, in accordance with the present invention, for parenteral administration, comprise sterile solutions, suspensions or emulsions, in an aqueous or non-aqueous solvent such as propylene glycol, polyethylene glycol or vegetable oils. These compositions may also contain coadjuvants, such as wetting, emulsioning and dispersing agents and preservatives. They can be sterilized by any known method or prepared as sterile solid compositions that will be dissolved in water or any other sterile injectable medium immediately before use. It is also possible to start with sterile raw materials and keep them in these conditions throughout the manufacturing process.

For rectal administration, the active ingredient may be formulated preferably as an oilbased suppository, such as for example vegetable oils or solid semi-synthetic glycerides, or in a hydrophilic base such as polyethylene glycols (macrogol).

The compounds of the invention can also be formulated for topical application for the treatment of pathologies in areas or organs accessible via this route, such as eyes, skin and intestinal tract. Formulations include creams, lotions, gels, powders, solutions and patches wherein the compound is found dispersed or dissolved in suitable excipients.

For nasal administration or by inhalation, the compound may be presented formulated in the form of an aerosol wherethrough it is conveniently released using suitable propellants.

The dosage and dosing frequency will vary in accordance, inter alia, with the nature and severity of the disease to be treated, the patient's age, general condition and weight, as well as with the particular compound administered and the administration route.

Throughout the description and the claims, the word "comprises" and its variants are not intended to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### DESCRIPTION OF THE FIGURES

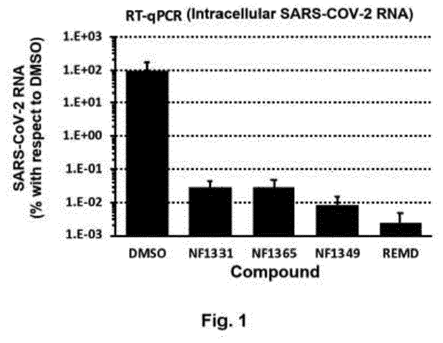

**Fig. 1****.** shows the reduction in intracellular RNA viral load in A549-ACE2 cells infected with SARS-CoV-2 in the presence of inhibitory concentrations of the compounds (25 µM) compared to cells treated with the reference compound Remdesivir (REMD; 5 µM).

### EXAMPLES

Next, the invention will be illustrated by means of assays carried out by the inventors.

### Example 1. Synthesis of compounds of the invention

According to the present invention, the compounds of formula **I** wherein R₁, R₂, and R₃ have the meanings defined in claim 1, can be prepared as described below, from commercially available products or from intermediates whose preparation is sufficiently described in the state of the art.

Compounds **NF1351, NF1353, NF1356, NF1349, NF1350, NF1352, NF1346, NF1322, NF1305, NF0818, NF1313, NF1316, NF1325, NF1355,** and **NF1354** were previously described (Conde, S.; Rodriguez-Franco, M. I.; Arce, M. P.; González-Muñoz, G. C.; Villarroya, M.; López, M. G.; Garcia, A. G. Novel dicarboxylic amino acid derivatives and the use thereof in the treatment of neurodegenerative diseases. WO 2009/074706 A1; Arce, M. P.; Rodriguez-Franco, M. I.; González-Muñoz, G. C.; Pérez, C.; López, B.; Villarroya, M.; López, M. G.; Garcia, A. G.; Conde, S. Neuroprotective and Cholinergic Properties of Multifunctional Glutamic Acid Derivatives for the Treatment of Alzheimer's Disease. J. Med. Chem. 2009, 52, 7249-7257; and Monjas, L.; Arce, M. P.; León, R.; Egea, J.; Pérez, C.; Villarroya, M.; López, M. G.; Gil, C.; Conde, S.; Rodriguez-Franco, M. I. Enzymatic and solid-phase synthesis of new donepezil-based L- and D-glutamic acid derivatives and their pharmacological evaluation in models related to Alzheimer's disease and cerebral ischemia. Eur. J. Med. Chem. 2017, 130, 60-72).

Compounds **NF1365, NF1331, NF1328, NF1363, NF1337, NF1334, NF1336, NF1358, NF1360,** and **NF1340** can be obtained by a solid-phase synthesis using NH₂-*L*-Glu[NH-2-(1-benzyl piperidin-4-yl)ethyl]-O-Wang resin as starting material, as explained below (Scheme 1).

In Scheme 1 the following abbreviations are used with the meanings shown below:
Ph represents a phenyl group,
EDC refers to *N*-ethyl-*N*'-(3-dimethylaminopropyl) carbodiimide,
HOBt refers to *N*-hydroxybenzotriazole,
Et₃N refers to trimethylamine,
DMAP refers to dimethylaminopyridine,
CH₂Cl₂ refers to dichloromethane,
DMF refers to dimethylformamide,
TFA is trifluoroacetic acid, and
SOCl₂ is thionyl chloride

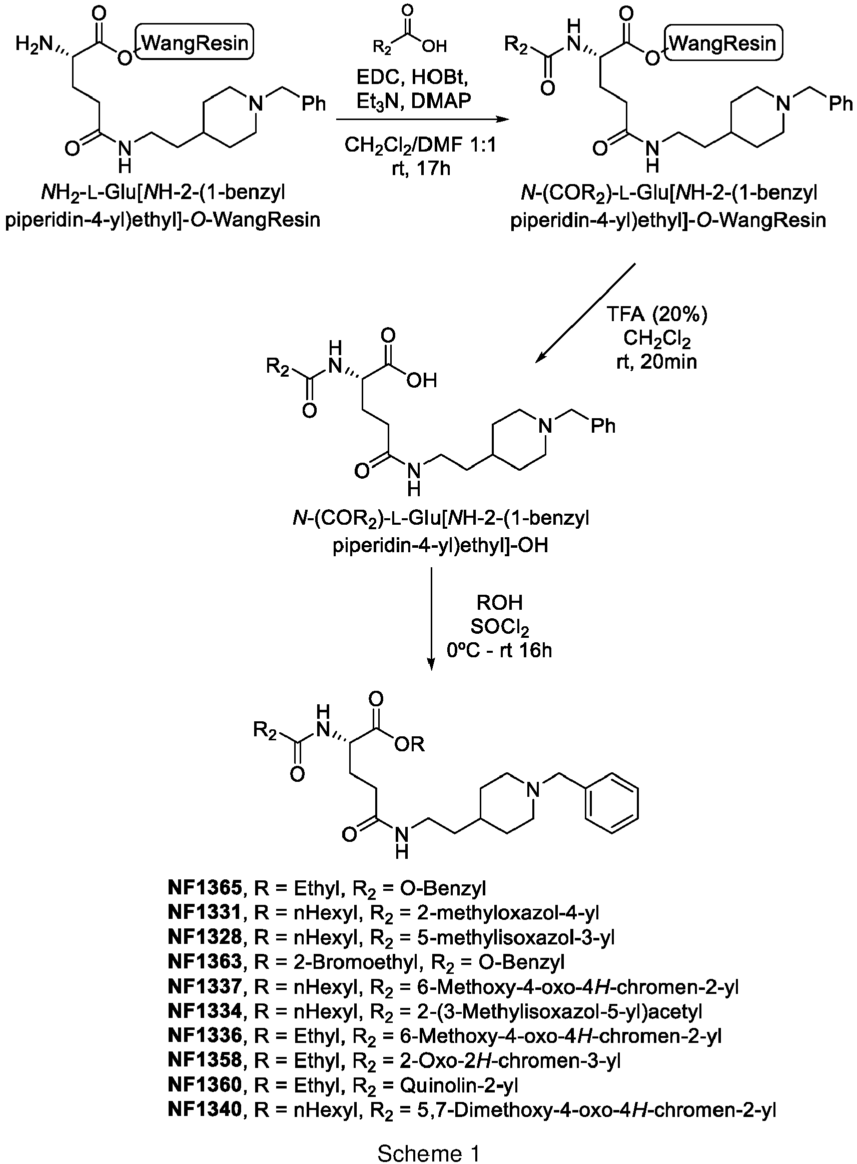

NH₂-*L*-Glu[NH-2-(1-benzylpiperidin-4-yl)ethyl]-*O*-WangResin, which was obtained according to (Eur. J. Med. Chem. 2017, 130, 60-72), was reacted at rt during 17 h with different aromatic and heteroaromatic carboxylic acids in the presence of EDC, HOBt, Et₃N, and DMAP, using in a mixture of CH₂Cl₂ and DMF (1:1) as solvent, to obtain intermediates *N*-(COR₂)-*L*-Glu[NH-2-(1-benzyl piperidin-4-yl)ethyl]-*O*-WangResin. The cleavage of the resin with trifluoroacetic acid (TFA) afforded the carboxylic acids N-(COR₂)-*L*-Glu[*N*H-2-(1-benzylpiperidin-4-yl)ethyl]-OH, which were then purified by automated flash column chromatography (Isolera-Biotage). The corresponding ethyl esters (**NF1365, NF1336, NF1358,** and **NF1360**), *n*-hexyl esters (**NF1331, NF1328, NF1337, NF1334, NF1340),** and 2-bromoethyl ester (**NF1363**) were obtained by reaction with ethanol, n-hexanol or 2-bromoethanol in the presence of thionyl chloride (SOCl₂).

### Example 1.1. Ethyl N²-((benzyloxy)carbonyl)-N⁵-(2-(1-benzylpiperidin-4-yl)ethyl)-L-glutaminate (NF1365)

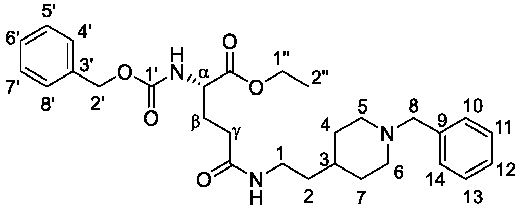

¹H-NMR (400 MHz, CDCl₃): δ 7.39-7.22 (m, 10H, H10-14, H4'-8'), 5.91 (m, 1H, NHCOCγ), 5.67 (d, 1H, *J* = 8.0 Hz, NHCα), 5.10 (s, 2H, H2'), 4.31 (m, 1H, Hα), 4.19 (q, 2H, *J* = 7.2 Hz, H1"), 3.49 (s, 2H, H8), 3.25 (m, 2H, H1), 2.87 (d, 2H, *J* = 10.8 Hz, H5_{eq},6_{eq}), 2.26-2.17 (m, 3H, 2Hγ, 1Hp), 1.94 (m, 3H, 1H_{β}, H5ₐₓ,6ₐₓ), 1.65 (m, 2H, H4_{eq},7_{eq}), 1.42 (m, 2H, H2), 1.28 (m, 3H, H3, H4ₐₓ,7ₐₓ), 1.24 (t, 3H, *J* = 7.2 Hz, H2"). ¹³C-NMR (100 MHz, CDCl₃): δ 172.1 (COCγ), 171.9 (COCα), 156.6 (C1'), 138.4 (C9), 136.4 (C3'), 129.5 (C4',8'), 128.8 (C10,14), 128.5 (C6'), 128.4 (C12'), 128.3 (C5',7'), 127.2 (C11,13), 67.3 (C2'), 63.6 (C8), 61.9 (C1"), 53.9 (C5,6), 53.8 (Cα), 37.6 (C1), 36.4 (C2), 33.7 (C3), 32.3 (C4,7), 31.2 (Cγ), 29.2 (Cβ), 14.4 (C2").

### Example 1.2. n-Hexyl N⁵-(2-(1-benzylpiperidin-4-yl)ethyl)-N²-(2-methyloxazole-4-carbonyl)-L-glutaminate (NF1331)

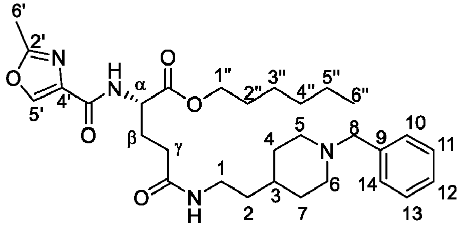

Melting point: 55-57 °C. [α]D²⁰: -7.3 (c= 1, CH₃OH). ¹H-NMR (400 MHz, CD₃OD): *δ* 8.28 (s, 1H, H5'), 7.45 (m, 5H, H10-14), 4.56 (m, 1H, Hα), 4.15 (m, 2H, H1"), 4.09 (s, 2H, H8), 3.21 (m, 4H, H1,5_{eq},6_{eq}), 2.76 (m, 2H, H5ₐₓ,6ₐₓ), 2.49 (s, 3H, H6'), 2.32 (m, 2H, Hγ), 2.27 (m, 1H, 1Hβ), 2.07 (m, 1H, 1Hβ), 1.90 (m, 2H, H4_{eq},7_{eq}), 1.65 (m, 2H, H2"), 1.56 (m, 1H, H3), 1.45 (m, 2H, H2), 1.42-1.26 (m, 8H, H4ₐₓ,7ₐₓ,3",4",5"), 0.89 (t, 3H, *J* = 7.1 Hz, H6"). ¹³C-NMR (100 MHz, CD₃OD): *δ* 174.5 (COC-γ), 172.9 (COC-α), 163.6 (C2'), 163.0 (CONHC-α), 142.9 (CH 5'), 136.8 (C 4'), 132.0 (2CH Ar), 130.5 (CH Ar), 130.4 (C Ar), 130.0 (2CH Ar), 66.6 (CH₂ 1"), 62.2 (CH₂ 8), 53.7 (2CH₂ 5, 6), 53.2 (CH α), 37.5 (CH₂ 1), 36.3 (CH₂ 2), 33.0 (CH₂ γ), 32.6 (CH₂ 3"), 32.5 (CH 3), 30.7 (2CH₂ 4,7), 29.6 (CH₂ 2"), 28.2 (CH₂ β), 26.6 (CH₂ 4"), 23.6 (CH₂ 5"), 14.3 (CH₂ 6"), 13.5 (CH₃ 6'). HRMS (ESI⁺): Calcd. for C₃₀H₄₅N₄O₅ [M+H]⁺ *m*/*z* 541.3384, found *m*/*z* 541.3393. HPLC-MS: t_{R} = 3.11 min (purity: 99 %).

### Example 1.3. n-Hexyl N⁵-(2-(1-benzylpiperidin-4-yl)ethyl)-N²-(5-methylisoxazole-3-carbonyl)-L-glutaminate (NF1328)

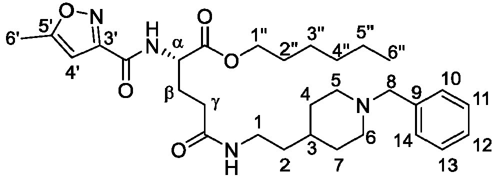

Melting point: 60-62 °C. [α]D²⁰: -10.4 (*c* = 1, CH₃OH). ¹H-NMR (400 MHz, CD₃OD): *δ*7.50 (m, 5H, H10-14), 6.48 (m, 1H, H4'), 4.56 (m, 1H, Hα), 4.30 (s, 2H, H8), 4.15 (m, 2H, H1"), 3.46 (m, 2H, H5eq, 6eq), 3.22 (m, 2H, H1), 3.03 (m, 2H, H5ax, 6ax), 2.49 (s, 3H, H6'), 2.37-2.26 (m, 3H, Hγ, 1Hβ), 2.07 (m, 1H, 1Hβ), 1.99 (m, 2H, H4eq, 7eq), 1.68-1.60 (m, 3H, H3, 2"), 1.46 (m, 2H, H2), 1.42-1.27 (m, 8H, H4ax, 7ax, 3", 4", 5"), 0.89 (t, *J* = 7.2 Hz, 3H, H6"). ¹³C-NMR (100 MHz, CD₃OD): *δ* 173.3 (COC-γ), 172.0 (C5'), 171.5 (COC-α), 160.5 (CONHC-α), 158.5 (C3'), 131.2 (2CH Ar), 130.0 (C Ar), 129.3 (CH Ar), 129.2 (2CH Ar), 100.8 (CH 4'), 65.5 (CH₂ 1"), 60.6 (CH₂ 8), 52.5 (2CH₂ 5, 6), 52.3 (CH α), 36.0 (CH₂ 1), 35.0 (CH₂ 2), 31.7 (CH₂ γ), 31.4 (CH₂ 3"), 30.9 (CH 3), 29.2 (2CH₂ 4, 7), 28.5 (CH₂ 2"), 26.5 (CH₂ β), 25.5 (CH₂ 4"), 22.4 (CH₂ 5"), 13.2 (CH₃ 6"), 10.9 (CH₃ 6'). HRMS (ESI⁺): Calcd. for C₃₀H₄₅N₄O₅ [M+H]⁺ 541.3384, found *m*/*z* 541.3379. HPLC-MS: t_{R} = 3.17 min (purity: 99 %).

### Example 1.4. 2-Bromoethyl N²-((benzyloxy)carbonyl)-N⁵-(2-(1-benzylpiperidin-4-yl)ethyl)-L-glutaminate (NF1363)

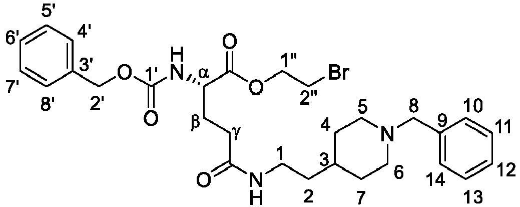

¹H-NMR (400 MHz, CDCl₃): δ 7.42-7.28 (m, 10H, H10-14, H4'-8'), 5.95 (m, 1H, NHCOCγ), 5.74 (d, 1H, *J* = 8.0 Hz, NHCα), 5.22 (s, 2H, H2'), 4.73 (m, 2H, H1"), 3.70 (m, 2H, H2"), 4.36 (m, 1H, Hα), 3.52 (s, 2H, H8), 3.26 (m, 2H, H1), 2.94 (d, 2H, *J* = 10.8 Hz, H5_{eq},6_{eq}), 2.28-2.17 (m, 3H, 2Hγ, 1H_{β}), 2.01 (m, 3H, 1H_{β}, H5ₐₓ,6ₐₓ), 1.85 (m, 2H, H4_{eq},7_{eq}), 1.68 (m, 2H, H2), 1.35 (m, 3H, H3, H4ₐₓ,7ₐₓ). ¹³C-NMR (100 MHz, CDCl₃): δ 172.8 (COCγ), 172.1 (COCα), 157.2 (C1'), 139.7 (C9), 138.5 (C3'), 130.4 (C4',8'), 127.4 (C10,14), 127.9 (C6'), 127.1 (C12'), 126.3 (C5',7'), 125.2 (C11,13), 67.5 (C1"), 67.1 (C2'), 64.6 (C8), 54.9 (C5,6), 53.4 (Cα), 37.8 (C1), 36.9 (C2), 34.8 (C3), 31.9 (C4,7), 30.6 (Cγ), 29.8 (C2"), 29.2 (Cβ).

### Example 1.5. n-Hexyl N⁵-(2-(1-benzylpiperidin-4-yl)ethyl)-N²-(6-methoxy-4-oxo-4H-chromene-2-carbonyl)-L-glutaminate (NF1337)

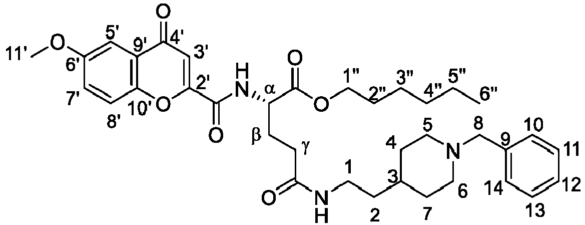

Melting point: 58-60 °C. [α]D²⁰: -11.0 (*c* = 1, CH₃OH). ¹H-NMR (500 MHz, CD₃OD): *δ*7.73 (d, *J* = 9.2 Hz, 1H, H8'), 7.52 (d, *J* = 3.1 Hz, 1H, H5'), 7.45 (dd, *J* = 9.2; 3.1 Hz, 1H, H7'), 7.34 (m, 5H, H10-14), 6.97 (s, 1H, H3'), 4.59 (m, 1H, Hα), 4.16 (m, 2H, H1"), 3.90 (s, 3H, H11'), 3.70 (s, 2H, H8), 3.20 (m, 2H, H1), 2.99 (m, 2H, H5eq, 6eq), 2.39-2.13 (m, 6H, Hγ, Hβ, H5ax, 6ax), 1.73 (m, 2H, H4eq, 7eq), 1.65 (m, 3H, H3, 2"), 1.44-1.20 (m, 10H, H2, 4ax, 7ax, 3", 4", 5"), 0.86 (t, *J* = 7.1 Hz, 3H, H6"). ¹³C-NMR (125 MHz, CD₃OD): *δ* 180.0 (CO 4'), 174.6 (COC-γ), 172.5 (COC-α), 161.5 (CONHC-α), 159.3 (C2'), 156.7 (C6'), 151.8 (C10'), 131.3 (2CH Ar), 129.6 (C Ar), 129.3 (CH Ar), 126.0 (2CH Ar), 125.9 (CH 7'), 125.0 (C9'), 121.4 (CH 8'), 111.3 (CH 3'), 105.7 (CH 5'), 69.1 (CH₂ 8), 66.7 (CH₂ 1"), 63.5 (CH₃ 11'), 56.5 (2CH₂ 5, 6), 54.2 (CH α), 37.8 (CH₂ 1), 36.7 (CH₂ 2), 33.6 (CH₂ γ), 33.1 (CH₂ 3"), 32.5 (CH 3), 31.9 (2CH₂ 4, 7), 29.6 (CH₂ 2"), 27.5 (CH₂ β), 26.7 (CH₂ 4"), 23.6 (CH₂ 5"), 14.6 (CH₃ 6"). HRMS (ESI⁺): Caldc. for C₃₆H₄₈N₃O₇ [M+H]⁺ 634.3487, found *m*/*z* 634.3490. HPLC-MS: t_{R}= 3.28 min (purity: 98 %).

### Example 1.6. n-Hexyl N⁵-(2-(1-benzylpiperidin-4-yl)ethyl)-N²-(2-(3-methylisoxazol-5-yl)acetyl)-L-glutaminate (NF1334)

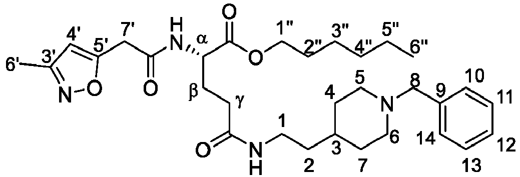

Melting point: 55-57 °C. [α]D²⁰: -14.1 (c = 0.8, CH₃OH). 1H-NMR (500 MHz, CD₃OD): δ 7.40 (m, 5H, H10-14), 6.21 (s, 1H, H4'), 4.37 (m, 1H, Hα), 4.12 (m, 2H, H1"), 3.90 (s, 2H, H8), 3.79 (s, 2H, H7'), 3.28-3.12 (m, 4H, H1 ,5_{eq},6_{eq}), 2.55 (m, 2H, H5ₐₓ,6ₐₓ), 2.29 (m, 2H, Hγ), 2.25 (s, 3H, H6'), 2.19 (m, 1H, 1Hβ), 1.93 (m, 1H, 1Hβ), 1.85 (m, 2H, H4_{eq},7_{eq}), 1.63 (m, 2H, H2"), 1.49 (m, 1H, H3), 1.47-1.27 (m, 10H, H2,4ₐₓ,7ₐₓ,3",4",5"), 0.90 (t, *J* = 7.1 Hz, 3H, H6"). ¹³C-NMR (125 MHz, CD₃OD): δ 174.4 (COC-γ), 173.1 (COC-α), 169.7 (CONHC-α), 168.2 (C5'), 161.7 (C3'), 131.8 (Ar), 130.0 (Ar), 129.9 (Ar), 105.2 (C4'), 66.6 (C1"), 62.9 (C8), 54.1 (C5,6), 53.7 (Cα), 37.6 (C1), 36.6 (C2), 34.7 (C7'), 33.1 (Cγ), 33.0 (C3"), 32.7 (C3), 31.4 (C4,7), 29.7 (C2"), 28.4 (Cβ), 26.7 (C4"), 23.7 (C5"), 14.5 (C6"), 11.3 (C6'). HRMS (ESI+): Calcd. for C₃₁H₄₇N₄O₅ [M+H]⁺ 555.3541, found *m*/*z* 555.3530. HPLC-MS: *t*_{R} = 3.08 min (purity: 98 %).

### Example 1.7. Ethyl N⁵-(2-(1-benzylpiperidin-4-yl)ethyl)-N²-(6-methoxy-4-oxo-4H-chromene-2-carbonyl)-L-glutaminate (NF1336)

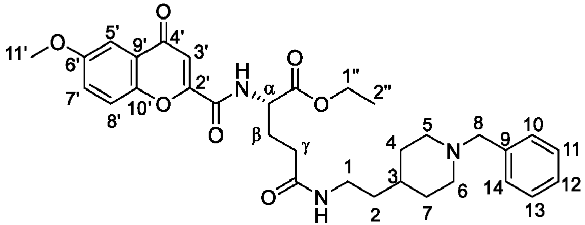

Melting point: 90-92 °C. [α]D²⁰: -12.1 (c= 1, CH₃OH). ¹H-NMR (500 MHz, CD₃OD): *δ*7.72 (d, *J* = 9.2 Hz, 1H, H8'), 7.52 (d, *J* = 3.1 Hz, 1H, H5'), 7.44 (dd, *J* = 9.2; 3.1 Hz, 1H, H7'), 7.37 (m, 5H, H10-14), 6.98 (s, 1H, H3'), 4.50 (m, 1H, Hα), 4.24 (m, 2H, H1"), 3.91 (s, 3H, H11'), 3.75 (s, 2H, H8), 3.22 (m, 2H, H1), 3.01 (m, 2H, H5_{eq},6_{eq}), 2.43-2.11 (m, 6H, Hγ,β,5ₐₓ,6ₐₓ), 1.82 (m, 2H, H4_{eq},7_{eq}), 1.65 (m, 1H, H3), 1.46-1.30 (m, 4H, H2,4ₐₓ,7ₐₓ), 1.28 (t, *J=* 7.1 Hz, 3H, H2"). ¹³C-NMR (125 MHz, CD₃OD): *δ* 180.0 (C4'), 175.1 (COC-γ), 172.1 (COC-α), 160.2 (CONHC-α), 158.6 (C2'), 156.9 (C6'), 150.8 (C10'), 131.3 (2CH Ar), 130.0 (C Ar), 129.5 (CH Ar), 127.3 (2CH Ar), 124.1 (C7'), 123.9 (C9'), 121.0 (C8'), 109.9 (C3'), 105.0 (C5'), 67.0 (C8), 60.6 (C1"), 57.3 (C11'), 55.6 (C5,6), 54.0 (Cα), 35.9 (C1), 35.0 (C2), 33.9 (Cγ), 32.0 (C3), 30.8 (C4,7), 27.1 (Cβ), 14.1 (C2"). HRMS (ESI⁺): Calcd. for C₃₂H₄₀N₃O₇ [M+H]⁺ 578.2861, found *m*/*z* 578.2888. HPLC-MS: *t*_{R} = 2.72 min (purity: 98 %).

### Example 1.8. Ethyl N⁵-(2-(1-benzylpiperidin-4-yl)ethyl)-N²-(2-oxo-2H-chromene-3-carbonyl)-L-glutaminate (NF1358)

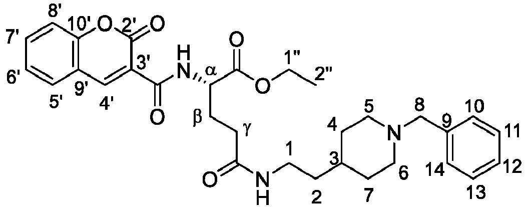

[α]D²⁰: -9.4 (c= 1, CH₃OH). ¹H-NMR (500 MHz, CD₃OD): *δ* 8.77 (s, 1H, H4'), 7.72 (d, 1H, *J* = 9.2 Hz, H5'), 7.55 (dt, 1H, *J* = 9.2; 1.9 Hz, H7'), 7.40 (m, 5H, H10-14), 7.37 (dd, 1H, *J* = 9.2; 1.9 Hz, H8'), 7.35 (dt,1H, *J* = 9.2; 1.9 Hz, H6'), 4.50 (m, 1H, Hα), 4.23 (m, 2H, H1"), 3.78 (s, 2H, H8), 3.22 (m, 2H, H1), 3.11 (m, 2H, H5_{eq},6_{eq}), 2.40-2.10 (m, 6H, Hγ,β,5ₐₓ,6ₐₓ), 1.85 (m, 2H, H4_{eq},7_{eq}), 1.72 (m, 1H, H3), 1.40-1.30 (m, 4H, H2,4ₐₓ,7ₐₓ), 1.28 (t, *J* = 7.1 Hz, 3H, H2"). ¹³C-NMR (125 MHz, CD₃OD): *δ* 176.3 (COC-γ), 171.8 (COC-α), 166.1 (CONHC-α), 162.1 (C2'), 153.7 (C10'), 132.5 (2CH), 131.1 (C4'), 130.0, 129.2, 128.7 (C7'), 128.6 (C5'), 127.0 (2C), 124.6 (C6'), 120.7 (C9'), 115.8 (C3'), 115.4 (C8'), 67.0 (C8), 60.6 (C1"), 55.6 (C5,6), 54.0 (Cα), 35.9 (C1), 35.0 (C2), 33.9 (C_{γ}), 32.0 (C3), 30.8 (C4,7), 27.1 (Cβ), 14.1 (C2").

### Example 1.9. Ethyl N⁵-(2-(1-benzylpiperidin-4-yl)ethyl)-N²-(quinoline-2-carbonyl)-L-glutaminate (NF1360)

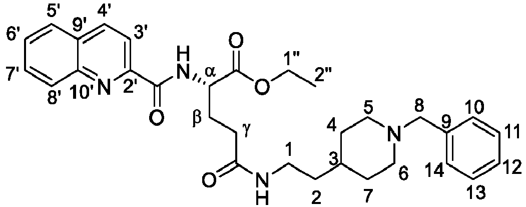

¹H-NMR (500 MHz, CD₃OD): *δ* 8.29 (dd, 1H, *J* = 8.9; 1.8 Hz, H4'), 8.21 (dd, 1H, *J* = 8.9; 1.8 Hz, H8'), 8.13 (d, 1H, *J* = 8.9 Hz, H3'), 7.90 (dt, 1H, *J* = 8.9; 1.8 Hz, H5') 7.86 (dt, 1H, *J* = 8.9; 1.8 Hz, H7'), 7.57 (dt, 1H, *J* = 8.9; 1.8 Hz H6'), 7.42 (m, 5H, H10-14), 4.62 (m, 1H, Hα), 4.43 (m, 2H, H1"), 3.89 (s, 2H, H8), 3.19 (m, 2H, H1), 3.10 (m, 2H, H5_{eq},6_{eq}), 2.30 (m, 6H, Hγ,β,5ₐₓ,6ₐₓ), 1.95 (m, 2H, H4_{eq},7_{eq}), 1.68 (m, 1H, H3), 1.35 (m, 4H, H2,4ₐₓ,7ₐₓ), 1.30 (t, *J* = 7.1 Hz, 3H, H2"). ¹³C-NMR (125 MHz, CD₃OD): *δ* 175.2 (COC-γ), 171.9 (COC-α), 164.9 (CONHC-α), 147.1 (C10'), 143.7 (C2'), 133.7 (2C), 131.2 (C4'), 130.2 (C7'), 129.8, 129.4 (C8'), 129.2, 128.6 (C9'), 127.4 (C5'), 127.2 (C6'), 126.5 (2C), 122.5 (C3'), 67.8 (C8), 61.4 (C1"), 56.8 (C5,6), 54.1 (Cα), 36.0 (C1), 35.2 (C2), 34.7 (C_{γ}), 32.4 (C3), 31.0 (C4,7), 28.2 (Cβ), 14.4 (C2").

### Example 1.10. n-Hexyl N⁵-(2-(1-benzylpiperidin-4-yl)ethyl)-N²-(5,7-dimethoxy-4-oxo-4H-chromene-2-carbonyl)-L-glutaminate (NF1340)

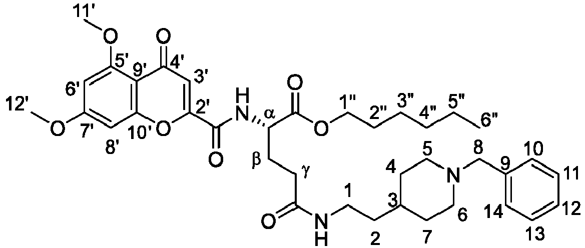

¹H-RMN (500 MHz, CD₃OD): δ 7.50 (m, 5H, H10-14), 6.85 (d, *J* = 2.3 Hz, 1H, H8'), 6.78 (s, 1H, H3'), 6.56 (d, *J* = 2.3 Hz, 1H, H6'), 4.58 (m, 1H, Hα), 4.29 (s, 2H, H8), 4.12 (m, 2H, H1"), 3.94 (s, 3H, H12'), 3.91 (s, 3H, H11'), 3.45 (m, 2H, H5_{eq}, 6_{eq}), 3.29-3.15 (m, 2H, H1), 3.00 (m, 2H, H5ₐₓ, 6ₐₓ), 2.43-2.31 (m, 3H, H_{γ}, 1Hβ), 2.16 (m, 1H, 1Hβ), 1.98 (m, 2H, H4_{eq},7_{eq}), 1.63 (m, 3H, H3,2"), 1.47-1.27 (m, 10H, H2,4ₐₓ,7ₐₓ,3",4",5"), 0.90 (t, 3H, *J* = 7.1 Hz, H6"). ¹³C-RMN (125 MHz, CD₃OD): δ 179.2 (CO 4'), 174.5 (COC-γ), 172.6 (COC-α), 167.2 (CONHC-α), 162.4 (C7'), 161.5 (C5'), 160.8 (C10'), 154.7 (C2'), 132.5 (2CH Ar), 131.3 (C Ar), 130.6 (CH Ar), 130.4 (2CH Ar), 113.9 (C3'), 110.2 (C9'), 98.0 (C6'), 94.7 (C8'), 66.6 (C1"), 62.9 (C8), 56.9 (C11'), 56.8 (C12'), 54.0 (C5,6), 53.9 (Cα), 37.4 (C1), 36.3 (C2), 33.8 (Cγ), 33.0 (C3"), 32.2 (C3), 30.5 (C4,7), 29.7 (C2"), 27.7 (Cβ), 26.7 (C4"), 23.7 (C5"), 14.5 (C6").

### Example 2: Protective effect against virus-induced cytopathic effect

Vero-E6 cells were seeded onto 96-well plates (2×10⁴ cells/well). Compounds were diluted from stock solutions in complete media (DMEM) supplemented with 10 mM HEPES, 1X non-essential amino acids (gibco), 100 U/ml penicillin-streptomycin (GIBCO) and 2 % fetal bovine serum (heat-inactivated at 56 °C for 30 min)) to achieve the desired concentration. A wide range of compound concentrations from 50 to 0.78 µM were tested.

SARS-CoV-2 stock strain NL/2020 (kindly provided by Dr. R. Molenkamp, Erasmus University Medical Center Rotterdam) was diluted to achieve a multiplicity of infection (MOI) of 0.001. Virus-compound mixture was used to inoculate the Vero-E6 monolayer. Cultures were maintained at 37 °C in a 5 % CO2 incubator for 72 h in the BSL3 facility. Cells were fixed using a 4 % formaldehyde-PBS solution to inactivate the virus. Virus-induced cell death was revealed by staining with a 0.1 % crystal violet solution in water-methanol for 30-60 minutes, time after which, plates were extensively washed with water and dried. Vehicle-treated (DMSO) infected- and uninfected-cells, were used as controls. Using crystal violet staining, the inventors determined the maximum (PCmax) and minimum (PCmin) dilutions of compounds capable of protecting the cell monolayer from virus-induced cell death. Approximate EC₅₀ values were estimated from these parameters for each of the compounds and are shown in Table 1.

An *in-silico* study of some ADME properties (SwissADME) predicted a high or medium gastrointestinal absorption in almost all cases, and absence of PAIN-like alerts in all derivatives (Table 1).

**Table 1**

| **Compound.** | **SARS-CoV-2 in Vero-E6 cells** | | | | | | ***In silico* GI Abs.** |
|---|---|---|---|---|---|---|---|
| | **PCmax** | **PCmin** | **PCmax real** | **PC min real** | **EC50 approx.** | **PCMAX/PC MIN ratio** | |
| **NF1365** | 50 | 3.125 | 51.0 | 3.2 | 2.4 | 16 | High |
| **NF1331** | 50 | 3.125 | 52.1 | 3.3 | 2.4 | 16 | High |
| **NF1351** | 50 | 6.25 | 45.9 | 5.7 | 4.3 | 8 | Medium |
| **NF1353** | 50 | 6.25 | 48.2 | 6.0 | 4.5 | 8 | Medium |
| **NF1356** | 50 | 6.25 | 48.5 | 6.1 | 4.5 | 8 | Medium |
| **NF1349** | 50 | 6.25 | 50.1 | 6.3 | 4.7 | 8 | High |
| **NF1328** | 50 | 6.25 | 52.1 | 6.5 | 4.9 | 8 | High |
| **NF1363** | 12.5 | 6.25 | 14.5 | 7.3 | 5.4 | 2 | High |
| **NF1350** | 50 | 6.25 | 73.5 | 9.2 | 6.9 | 8 | High |
| **NF1337** | 50 | 12.5 | 41.0 | 10.3 | 7.7 | 4 | Medium |
| **NF1352** | 25 | 12.5 | 24.5 | 12.3 | 9.2 | 2 | Medium |
| **NF1346** | 50 | 12.5 | 49.5 | 12.4 | 9.3 | 4 | High |
| **NF1322** | 50 | 12.5 | 50.0 | 12.5 | 9.4 | 4 | High |
| **NF1305** | 50 | 12.5 | 50.1 | 12.5 | 9.4 | 4 | High |
| **NF0818** | 50 | 12.5 | 51.9 | 13.0 | 9.7 | 4 | High |
| **NF1334** | 50 | 25 | 27.9 | 14.0 | 10.5 | 2 | High |
| **NF1336** | 50 | 25 | 49.0 | 24.5 | 18.4 | 2 | High |
| **NF1313** | 50 | 25 | 50.7 | 25.3 | 19.0 | 2 | High |
| **NF1358** | 50 | 25 | 50.8 | 25.4 | 19.1 | 2 | High |
| **NF1316** | 50 | 25 | 51.8 | 25.9 | 19.4 | 2 | High |
| **NF1325** | 50 | 25 | 52.2 | 26.1 | 19.6 | 2 | High |
| **NF1355** | 50 | 25 | 52.4 | 26.2 | 19.7 | 2 | Low |
| **NF1354** | 25 | 25 | 27.8 | 27.8 | 20.9 | 1 | Low |
| **NF1360** | 50 | 50 | 32.3 | 32.3 | 24.3 | 1 | High |
| **NF1340** | 50 | 50 | 55.7 | 55.7 | 41.8 | 1 | Low |

### Example 3: Intracellular viral antigen accumulation

To confirm that the monolayer protection effect displayed by the identified compounds was indeed directly related to an antiviral effect and not to an unrelated effect, Vero-E6 and A549-ACE2 cell monolayers were inoculated at MOI 0.01 or 0.005, respectively, in the presence of non-toxic compound concentrations. Twenty-four (forty-eight for infections performed in A549-ACE2) hours later cells were fixed by 20 minutes incubation with a 4 % formaldehyde-PBS solution. After extensive washes with PBS, infection efficiency was evaluated by the detection of intracellular SARS-CoV-2 N protein accumulation by immunofluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated (DMSO) conditions was used as reference (100 %). EC₅₀ and EC₉₀ values were calculated from normalized data and are shown in Tables 2 and 3. Number of nuclei in vehicle-treated (DMSO) conditions was used as reference (100 %) to calculate the CC₅₀ (DAPI) values.

### Example 4: Determination of cytotoxicity by MTT colorimetric assay:

To evaluate the cytotoxicity profile of the selected compounds MTT assays were performed in Vero-E6 and A549-ACE2 cells. Uninfected cells were incubated with a range of compound concentrations (from 50 to 0.78 µM) for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated (DMSO) cells were used as reference (100 %). CC₅₀ values were calculated from normalized data and are shown in Tables 2 and 3.

**Table 2**

| | **A549-ACE2 (human lung cell line)*** | | | |
|---|---|---|---|---|
| **Compound** | **EC₅₀** | **EC₉₀** | **CC₅₀ (DAPI)** | **CC₅₀ (MTT)** |
| **NF1331** | 1.8 | 5 | >50 | >50 |
| **NF1365** | 1 - 2 | 2.5 - 5 | 25 | 45 |
| **NF1349** | 3 | 5.5 | 30 | 48 |

| | | | | |
|---|---|---|---|---|
| * Values expressed in micromolar | | | | |

**Table 3**

| | **Vero-E6 (African green line)* monkey kidney cell** | | | |
|---|---|---|---|---|
| **Compound** | **EC₅₀** | **EC₉₀** | **CC₅₀ (DAPI)** | **CC₅₀ (MTT)** |
| **NF1331** | 1.5 | 11 | >50 | >50 |
| **NF1365** | 1.8 | 6 | 50 | >50 |
| **NF1349** | 3.1 | 7 | >50 | >50 |

| | | | | |
|---|---|---|---|---|
| * Values expressed in micromolar | | | | |

In general, it is observed that the antiviral activity of the compounds is maintained (EC50 and EC90) in the same concentration range in A549-ACE2 cells compared to the indexes in Vero-E6 cells. Furthermore, in terms of the cytotoxicity indexes (CC50), the analysis of the number of cells (dapi) present at the end of the study in the infected cultures, and of mitochondrial activity (MTT) in uninfected cultures treated in parallel, demonstrate that in general these compounds are hardly toxic to cells and that, therefore, the effect of reducing the accumulation of the viral protein (read out of the antiviral assay) is due to a genuine antiviral effect and not a consequence of apparent toxic effects. These results suggest that the compounds present an acceptable therapeutic window at least in cell culture.

### Example 5: Intracellular viral RNA quantitation

To analyze the maximum impact on virus propagation, A549-ACE2 cell monolayers were inoculated at MOI 0.001 in the presence of non-toxic compound concentrations (25 mM). Forty-eight hours later cell lysates were prepared using Trizol reagent (Thermo Scientific) and the viral RNA content was determined by RT-qPCR using previously validated sets of primers and probes specific for the detection of the SARS-CoV-2 E gene and the cellular 18S gene, for normalization purposes. ΔCt method was used for relative quantitation of the intracellular viral RNA accumulation in compound-treated cells compared to the levels in infected cells treated with DMSO, set as 100 %. Results that are summarized in Figure 1 demonstrate that compounds produce up to 4 log reduction in viral RNA load, similar to the effect of remdesivir (REMD at 5 µM).

### Example 6: Analysis of the effect on HCoV 229E infection

To study the antiviral effect of selected compounds of the family against a distinct human coronavirus, Huh-7 cell monolayers were infected with a GFP-expressing recombinant HCoV 229E (229E-GFP) at MOI 0.01 in the presence of a wide range of compound concentrations (0.4 to 50 µM). Forty-eight hours later cells were fixed by 10 minutes incubation with a 4 % formaldehyde-PBS solution. After extensive washes with PBS, infection efficiency was evaluated by the detection of GFP accumulation by fluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated (DMSO) conditions was used as reference (100 %). EC₅₀ and EC₉₀ values were calculated from normalized data and are shown in Table 4.

Cytotoxicity profile of the selected compounds was evaluated by MTT assays in parallel wells of Huh-7 cells. Uninfected cells were incubated with a range of compound concentrations (from 50 to 0.78 µM) for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated (DMSO) cells were used as reference (100 %). CC₅₀ values were calculated from normalized data and are shown in Table 4.

Results showed that compound concentrations that inhibit SARS-CoV-2 propagation are also able to control 229E-GFP infection suggesting that compounds display a pan-coronavirus antiviral activity.

**Table 4**

| **229E-GFP** | **Huh-7 (human hepatic cell line)*** | | | |
|---|---|---|---|---|
| **Compound** | **EC₅₀** | **EC₉₀** | **CC₅₀ (DAPI)** | **CC₅₀ (MTT)** |
| **NF1331** | 2 | 20 | 43 | >50 |
| **NF1365** | 1 | 8 | 28 | 24 |
| **NF1349** | 2 | 10 | 30 | 28 |

| | | | | |
|---|---|---|---|---|
| * Values expressed in micromolar | | | | |

### Example 7: Analysis of the effect on VSV and WNV infection

To characterize the antiviral spectrum of selected compounds of the family, A549-ACE2 cell monolayers were infected with a GFP-expressing recombinant vesicular stomatitis virus (VSV-GFP) at MOI 0.01 in the presence of a wide range of compound concentrations (0.4 to 50 µM). Sixteen hours later cells were fixed by 20 minutes incubation with a 4 % formaldehyde-PBS solution. After extensive washes with PBS, infection efficiency was evaluated by the detection of GFP accumulation by fluorescence microscopy. Nuclei were stained using DAPI (4',6-diamidino-2-phenylindole) dye. Automated imaging was carried out in a SparkCyto (Tecan) multimode plate reader. The total intensity signal present in vehicle treated (DMSO) conditions was used as reference (100 %). EC₅₀ and EC₉₀ values were calculated from normalized data and are shown in Table 5. Results showed that compound concentrations that inhibit SARS-CoV-2 propagation are not able to control VSV-GFP infection suggesting that compounds display a selective antiviral activity towards SARS-CoV-2.

**Table 5**

| **VSV-GFP** | **A549-ACE2 (human lung cell line)*** | | | |
|---|---|---|---|---|
| **Compound** | **EC₅₀** | **EC₉₀** | **CC₅₀ (dapi)** | **CC₅₀ (MTT)** |
| **NF1331** | 38 | >50 | >50 | >50 |
| **NF1365** | 36 | 47 | 25 | 45 |
| **NF1349** | 36 | 47 | 30 | 48 |

| | | | | |
|---|---|---|---|---|
| * Values expressed in micromolar | | | | |

These results were extended using West Nile virus, a positive stranded RNA virus of the Flaviviridae family of viruses. Huh-7 cell monolayers were infected with a GFP-expressing recombinant West Nile virus (WNV-GFP) at MOI 0.01 in the presence of a wide range of compound concentrations (0.4 to 50 µM). Forty-eight hours later cells were fixed by 20 minutes incubation with a 4 % formaldehyde-PBS solution. After extensive washes with PBS, infection efficiency was evaluated by the detection of GFP accumulation by fluorescence microscopy. Nuclei were stained using DAPI (4',6-diamidino-2-phenylindole) dye. Automated imaging was carried out in a SparkCyto (Tecan) multimode plate reader. The total intensity signal present in vehicle treated (DMSO) conditions was used as reference (100 %). EC₅₀ and EC₉₀ values were calculated from normalized data and are shown in Table 6. Results showed that compound concentrations that inhibit HCoV 229E-GFP propagation are not able to control WNV-GFP infection in the same cell line suggesting that compounds display a selective antiviral activity towards coronaviruses.

**Table 6**

| **WNV-GFP** | **Huh-7 human hepatic cell line)*** | | | |
|---|---|---|---|---|
| **Compound** | **EC₅₀** | **EC₉₀** | **CC₅₀ (dapi)** | **CC₅₀ (MTT)** |
| **NF1331** | >50 | >50 | >50 | >50 |
| **NF1365** | 27 | 45 | 30 | 24 |
| **NF1349** | 36 | >50 | 38 | 28 |

| | | | | |
|---|---|---|---|---|
| * Values expressed in micromolar | | | | |

Collectively the results in examples 2 to 6 demonstrate that compounds of the invention display selective antiviral activity towards members of the *Coronaviridae* family of viruses, such as SARS-CoV-2 and HCoV 229E.

## Claims

1. A compound of formula **I** 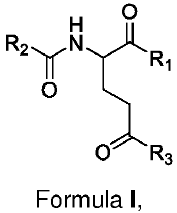 or a pharmaceutically acceptable salt thereof, wherein:
R₁ represents an -OH, -OR₄ or -NHR₅ group;
R₂ represents a C₁-C₆ alkyl group optionally substituted by phenyl, an -OCy₁ or Cy₁ group;
R₃ represents an -OH, -OR₄ or -NHR₅ group;
R₄ represents a C₁-C₆ alkyl optionally substituted by a halogen group;
R₅ represents a C₁-C₆ alkyl optionally substituted by a Cy₂ group;
Cy₁ represents a 5 or 6-membered aromatic ring that is optionally fused to another 5 or
6-membered aromatic ring and optionally contains 1 or 2 heteroatoms selected from N,
O and S, and is optionally substituted by one or more C₁-C₆ alkyl or -O-C₁-C₆ alkyl groups; and
Cy₂ represents a 5 or 6 membered saturated heterocycle containing 1 or 2 heteroatoms selected from N, O and S, and is optionally substituted by a C₁-C₆ alkyl group which in turn is optionally substituted by a phenyl group,
for use in the treatment and/or prevention of a viral disease caused by a coronavirus.

2. The compound of formula **I** for use according to claim 1, wherein the coronavirus is SARS-CoV-2, HCoV 229E, HCoV NL63, HCoV HKU1, HCoV OC43, SARS-CoV-1 or MERS-CoV.

3. The compound of formula I for the use according to claim 2, wherein the coronavirus is SARS-CoV-2 or HCoV 229E.

4. The compound of formula I for the use according to any of claims 1 to 3, wherein R₁ represents an -OR₄ group.

5. The compound of formula **I** for the use according to any of claims 1 to 4, wherein R₄ represents a C₁-C₆ alkyl optionally substituted by a Br group.

6. The compound of formula **I** for the use according to any of claims 1 to 5, wherein R₂ represents a C₁-C₆ alkyl group substituted by phenyl, an -OCy₁ or Cy₁ group.

7. The compound of formula **I** for the use according to any of claims 1 to 6, wherein R₃ represents an -NHR₅ group.

8. The compound of formula **I** for the use according to any of claims 1 to 7, wherein R₅ represents a C₁-C₆ alkyl optionally substituted by a Cy₂ group.

9. The compound of formula **I** for the use according to any of claims 1 to 8, wherein Cy₂ represents a heterocycle of formula Cy₂ₐ:

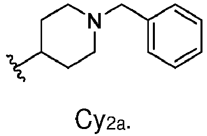

10. The compound of formula **I** for the use according to claim 1, wherein:
R₁ represents an -OR₄ group;
R₂ represents a C₁-C₆ alkyl group substituted by phenyl, an -OCy₁ or Cy₁ group;
R₃ represents an -NHR₅ group;
R₄ represents a C₁-C₆ alkyl which is optionally substituted by a halogen group.

11. The compound of formula **I** for the use according to claim 1, wherein the compound of formula **I** is selected from:

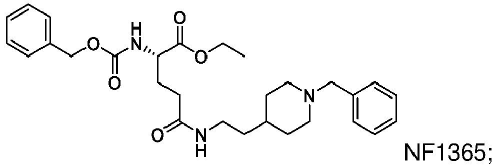

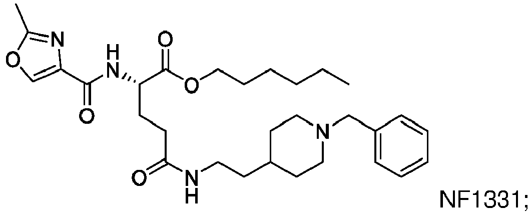

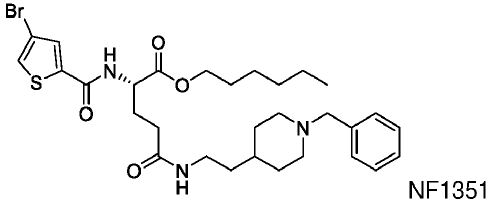

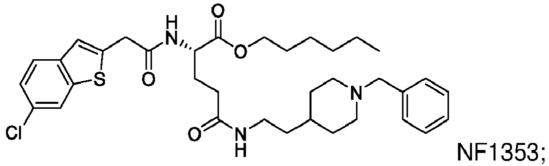

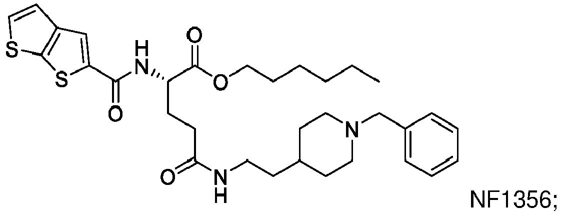

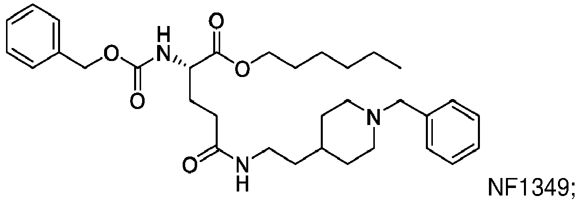

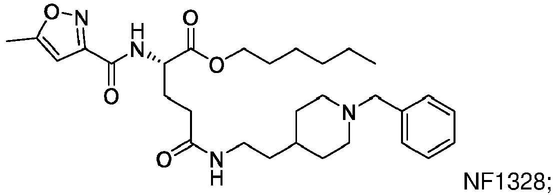

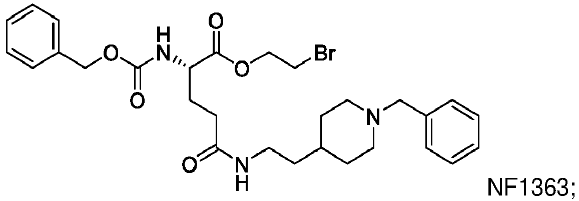

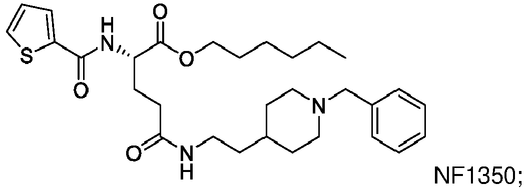

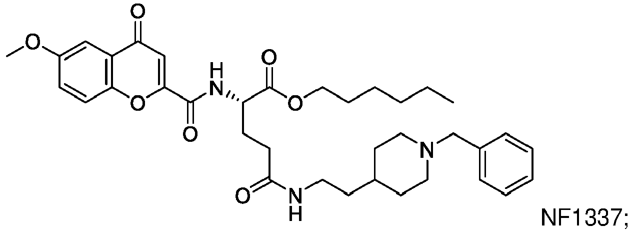

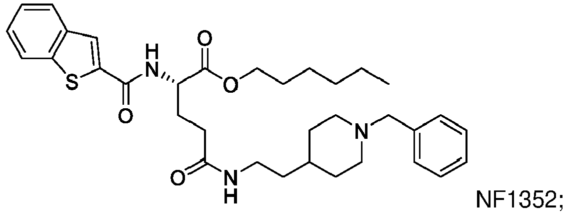

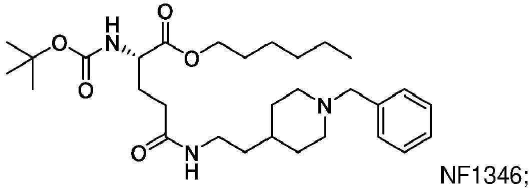

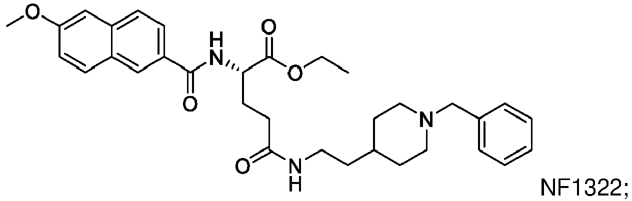

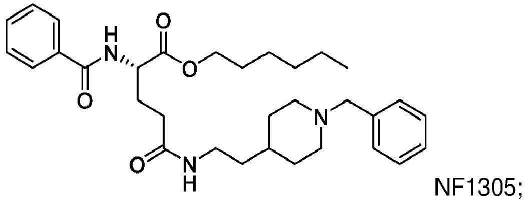

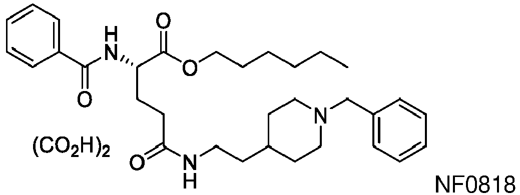

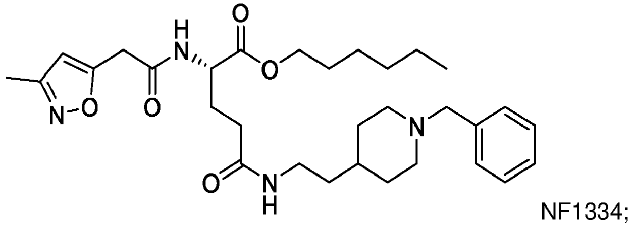

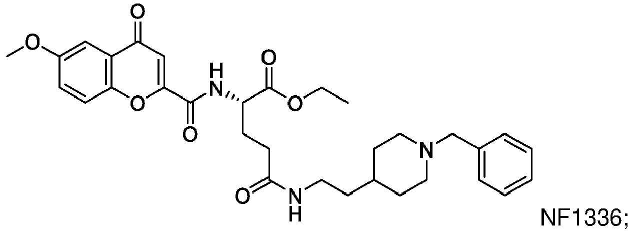

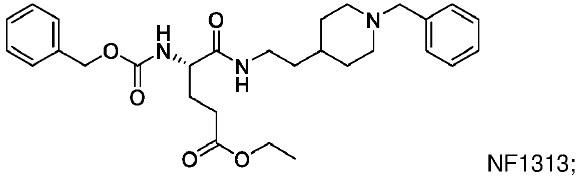

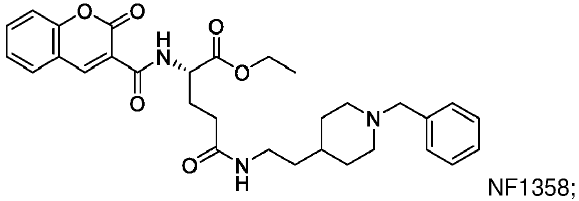

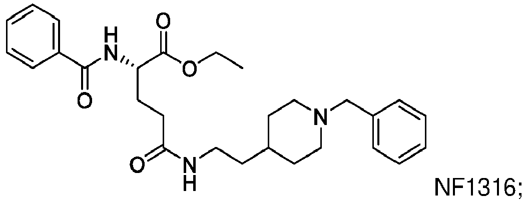

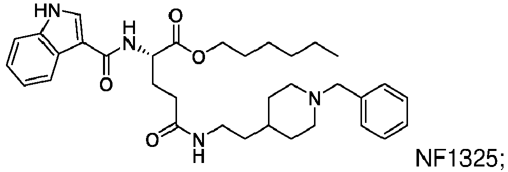

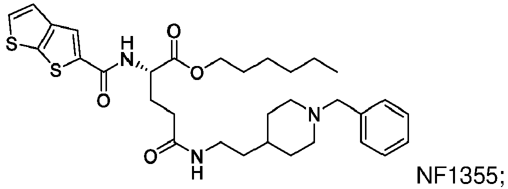

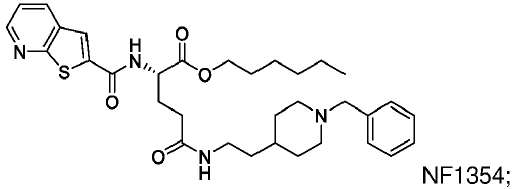

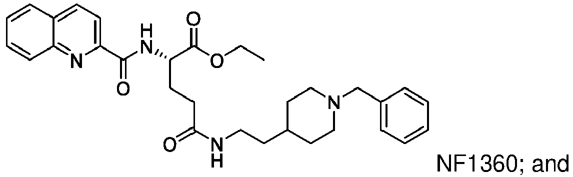

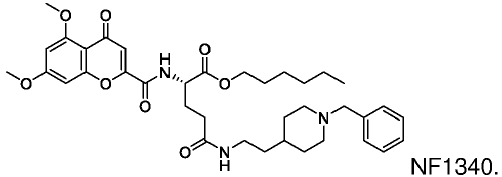

12. The compound of formula **I** for the use according to claim 11, wherein the compound of formula **I** is selected from NF1331, NF1365 and NF1349.

13. A compound of formula **I** selected from: 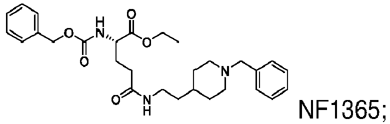 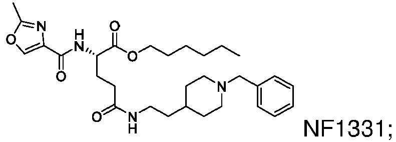 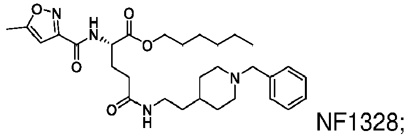 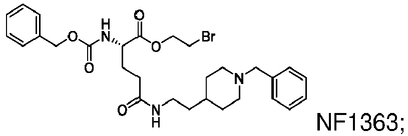 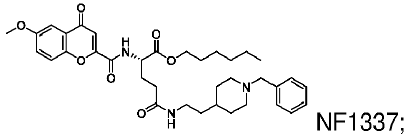 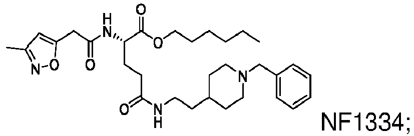 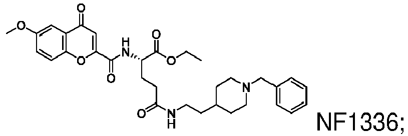 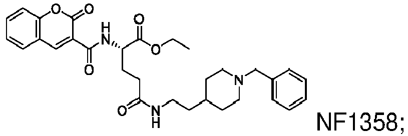 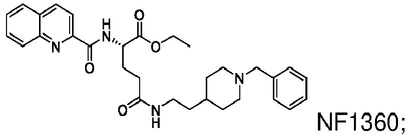 and

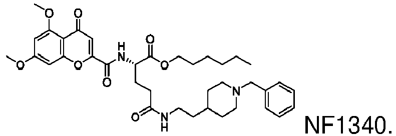

14. A compound of formula **I** selected from: 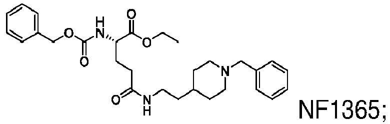 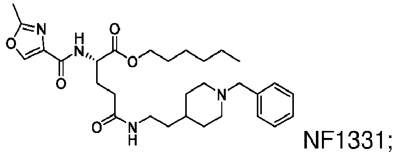 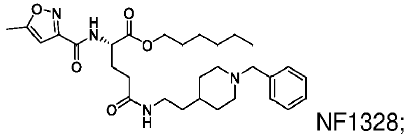 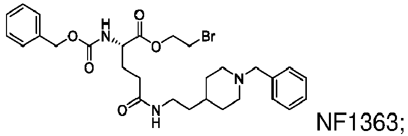 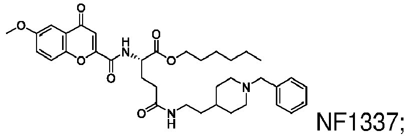 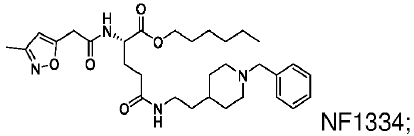 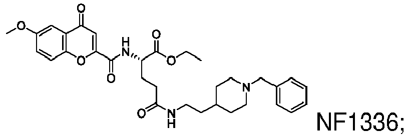 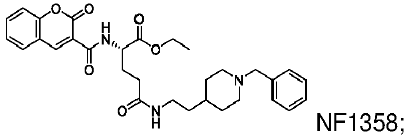 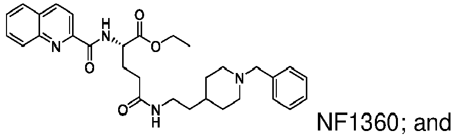 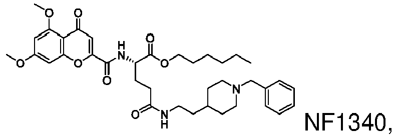 or a pharmaceutically acceptable salt thereof for use as a medicament.
